# EUROPEAN PATENT APPLICATION

(11) **EP 4 834 929 A1**
(43) Date of publication of application: **04.11.2026**
(21) Application number: 25173504.9
(22) Date of filing: 30.04.2025
(51) Int. Cl.: C07K 16/00, C07K 16/06

(54) **METHODS FOR THE PRODUCTION OF HETERODIMERIC POLYPEPTIDES**

(71) Applicant: Gyes B.V., 3584 CM Utrecht (NL)
(72) Inventor: Parren, Paul Willem Henri Ida, 3584 CM Utrecht (NL); Oostindie, Simone Charlotte, 3584 CM Utrecht (NL); Lutje Hulsik, David, 3584 CM Utrecht (NL); Logtenberg, Ton, 3584 CM Utrecht (NL)
(74) Representative: Kirkpatrick

(57) **Abstract**

The invention relates to a method for producing a heterodimeric polypeptide composition, said heterodimeric polypeptide comprising a first polypeptide and a second polypeptide, wherein the first polypeptide comprises an N- or C-terminal histidine-based tag that more efficiently binds to a histidine affinity resin when present on the termini of both polypeptides of a dimer than when present on the terminus of only one polypeptide of a dimer, said method comprising: providing a composition comprising heterodimers of the first and second polypeptides and homodimers of the first polypeptide; contacting said composition with a histidine affinity resin and allowing homodimers of the first polypeptide to preferentially bind to the resin, and recovering the unbound fraction to obtain a composition that is enriched for heterodimers.

## Description

### FIELD OF THE INVENTION

The present invention relates to the production and purification of heteromeric proteins, in particular heterodimeric antibodies, such as bispecific or trispecific antibodies.

### BACKGROUND OF THE INVENTION

A challenge in the production of heterodimeric antibodies, such as bispecific antibodies, is that two or more monomeric heavy chain polypeptides can form both heterodimers as well as homodimers. Thus, recombinant production of heterodimeric antibodies in a single cell typically yields homodimers of each of two different polypeptides in addition to the desired heterodimer. Similar challenges may occur in the production of other types of heterodimeric proteins based on subunits that are also capable of forming homodimers.

In the field of heterodimeric antibodies, the production of undesired homodimers can be reduced by introducing asymmetric amino acid mutations in the two monomers that favor the formation of heterodimers over the formation of homodimers. Well-known examples of such asymmetric amino acid mutations are the knob-in-hole mutations (Ridgway et al. (1996) Protein Eng. 9(7):617-21), the DuoBody^{®} mutations (Labrijn et al. (2013) Proc Natl Acad Sci U S A. 110(13):5145-50) and the introduction of asymmetric charged residues (De Nardis et al (2017) J Biol Chem. 292(35):14706-14717).

However, these asymmetric amino acid mutations typically do not prevent formation of homodimers entirely and production of heterodimeric antibodies in a single cell (or in vitro by controlled Fab-arm exchange (Labrijn et al. (2013) Proc Natl Acad Sci U S A. 110(13):5145-50) typically yields a product that still contains a significant amount of one or both homodimers. This is particularly the case if one of the two homodimers have discordant expression levels, or if, in post-production

Fab-arm exchange, one of the homodimers is added in excess.

Various purification techniques, such as chromatography-based techniques, may be used to separate the desired heterodimer from undesired homodimers. These may be based on capturing heterodimers and wash away homodimers and other impurities, or be based on capturing homodimers to remove them.

Liang et al. (2023) J Chromatography B, 1225, 123767 describe removal of homodimers by using weak partitioning mode anion exchange chromatography. The method can be used to remove negatively-charged homodimers.

Production of libraries of heterodimeric proteins, such as bispecific antibodies, involves producing many small samples of heterodimers in parallel and in high-throughput. In the functional screening of bispecific antibody libraries, the presence of significant amounts of homodimer is undesirable as the homodimer may interfere with the functional read-out.

While several methods of separating heterodimers and homodimers are known in the art, these are often designed for large scale manufacturing of a single bispecific antibody product and are often laborious and not suitable for high-throughput screening.

There is thus a need for robust, easy and generally applicable methods for removing undesired homodimers from a mixture of heterodimers and homodimers in order to enrich for the desired heterodimer in the supernatant.

### SUMMARY OF THE INVENTION

In a first aspect, the invention relates to a method for producing a heterodimeric polypeptide composition, said heterodimeric polypeptide comprising a first polypeptide and a second polypeptide,
wherein said first polypeptide and said second polypeptide are different and are able to form heterodimers and homodimers,
and wherein the first polypeptide comprises an N- or C-terminal histidine-based tag that more efficiently binds to a histidine affinity resin when present on the termini of both polypeptides of a dimer than when present on the terminus of only one polypeptide of a dimer, said method comprising:
   a) providing a composition comprising heterodimers of the first and second polypeptides and homodimers of the first polypeptide, preferably by:
      (i) co-expressing said first polypeptide and second polypeptides in a single host cell, wherein preferably the first polypeptide is overexpressed relative to the second polypeptide, and collecting supernatant,
         or
      (ii) expressing said first polypeptide and second polypeptide in separate host cells, collecting and mixing supernatants, wherein preferably the first polypeptide is added in excess, and performing an exchange reaction to obtain heterodimers,
   b) contacting said composition with a histidine affinity resin and allowing homodimers of the first polypeptide to preferentially bind to the resin, and
   c) recovering the unbound fraction to obtain a composition that is enriched for heterodimers.

In a further aspect, the invention relates to a composition obtainable by the method of the invention as described herein.

In a further aspect, the invention relates to a method for producing and screening a library comprising a plurality of heterodimeric polypeptides, comprising performing the method according to the invention for a plurality of heterodimeric polypeptides in parallel and performing the further step of screening the plurality of compositions obtained in step c) for heterodimers having desired functional properties.

### DETAILED DESCRIPTION OF THE FIGURES

Figure 1: Analysis of the preparation of CSR-Fab-hIgG4-R409K-F405Lxgp120-VHH-hIgG4-G4-H6 using 5 µL beads, 16 hour roller bench incubation.
Figure 2: Schematic representation of the resulting antibody heterodimer.
Figure 3: LabChip analysis of 3 of the produced antibody preparations before (pre) and after (post) purification.

### DETAILED DESCRIPTION OF THE INVENTION

The term "immunoglobulin" as used herein is intended to refer to a class of structurally related glycoproteins typically consisting of two pairs of polypeptide chains, one pair of light (L) chains and one pair of heavy (H) chains, all four potentially inter-connected by disulfide bonds, although some mammalian species also produce heavy-chain only antibodies. The term "immunoglobulin heavy chain", "heavy chain of an immunoglobulin" or "heavy chain" as used herein is intended to refer to one of the chains of an immunoglobulin. A heavy chain is typically comprised of a heavy chain variable domain (abbreviated herein as VH) and a heavy chain constant domain (abbreviated herein as CH) which defines the isotype of the immunoglobulin. The heavy chain constant domain typically is comprised of three domains, CH1, CH2, and CH3. The heavy chain constant domain further comprises a hinge domain. Within the structure of the immunoglobulin (e.g. IgG), the two heavy chains are inter-connected via disulfide bonds in the hinge region. Equally to the heavy chains, each light chain is typically comprised of several domains: a light chain variable domain (VL) and a light chain constant domain (CL). Furthermore, the VH and VL domains may be subdivided into regions of hypervariability (or hypervariable regions which may be hypervariable in sequence and/or form structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4. CDR sequences may be determined by use of various methods, e.g. the methods provided by Kabat et al. (1991) Sequence of protein of immunological interest, fifth edition. NIH publication or Choitia and Lesk (1987) J. Mol. Biol. 196:901. Various methods for CDR determination and amino acid numbering can be compared on www.abysis.org (UCL).

The term "antibody" is intended to refer to an immunoglobulin molecule (including, as explained below, fragments thereof such as Fabs or VHH-based constructs), comprising one or more antigen-binding regions, that have an ability to specifically bind to an antigen under typical physiological conditions with a half-life of significant periods of time, such as at least about 30 minutes, at least about one hour, at least about two hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). In combination with physiologically relevant on-rates, such binding half-lives are a characteristic of medium or high affinity antigen-binding regions (e.g. those with affinities having a K_{D} of 10⁻⁷ M or less, such as between 10⁻¹² and 10⁻⁷ M). Bi- or multispecific antibodies may, in some embodiments, comprise multiple antigen-binding regions in which medium/high affinity antigen-binding regions are combined with low affinity antigen-binding regions. Such low affinity antigen-binding regions being characterized by affinities having a K_{D} of 10⁻⁷ M or greater, such as between 10⁻⁷ and 10⁻⁴ M. Antigen-binding regions which interact with an antigen may comprise variable domains of both the heavy and light chains of an immunoglobulin molecule or may comprise or consist of single-domain antigen-binding regions, for example a heavy chain- or light chain variable domain only. The constant regions of an antibody, if present, may mediate the binding of the immunoglobulin to host tissues or factors, including various cells of the immune system (such as effector cells and regulatory cells) and components of the complement system such as C1q, the first component in the classical pathway of complement activation. In some embodiments, however, the Fc region of the antibody has been modified to become inert, "inert" means an Fc region which is at least not able to, or has strongly reduced ability to, bind any Fc receptor, such as any Fcy receptor, induce Fc-mediated cross-linking of FcRs, or induce FcR-mediated cross-linking of target antigens via two Fc regions of individual antibodies. In a further embodiment, the inert Fc region is in addition not able to bind C1q.

The Fc region of an immunoglobulin is defined as the fragment of an antibody which would be typically generated after digestion of an antibody with papain which includes the two CH2-CH3 domains of an immunoglobulin and a connecting region, e.g. a (partial) hinge region, or structurally equivalent thereof. The constant domain of an antibody heavy chain defines the antibody isotype, e.g. IgG1, IgG2, IgG3, IgG4, IgA1, IgA2, IgM, IgD, or IgE. The Fc-region mediates the effector functions of antibodies with cell surface receptors called Fc receptors and proteins of the complement system.

The term "Fab region" or "Fab fragment" refers to a region composed of a variable and a constant domain of each of the heavy and the light chain of an antibody. Papain cleavage of a antibody generates an Fc region and two Fab regions.

The term "hinge region" as used herein is intended to refer to the hinge region of an immunoglobulin heavy chain. Thus, for example, the hinge region of a human IgG1 antibody corresponds to amino acids 216-238 according to the EU numbering. The hinge region consists of two interconnected hinge domains, which are further subdivided into an upper hinge domain, a core hinge domain and a lower hinge domain. The upper, core and lower hinge domain of wild-type IgGs (listed as SEQ ID NO:1-11 in the sequence listing) are defined according to the following table from a review article by Chiu et al. (2019) Antibodies 8, 55; doi: 10.3390, wherein the numbers "216", "226" and "231" refer to the positions in IgG1 according to Eu numbering:

| IgG Hinge Sequences | | | |
|---|---|---|---|
| Isotype | Upper | Core | Lower |
| | 2 | 2 | 2 |
| | 1 | 2 | 3 |
| | 6 | 6 | 1 |
| *IgG1* | EPKSCDKTHT | CPPCP | APELLGGP |
| *IgG2* | ERK | CCVECPPCP | APPVA GP |
| *IgG3* | ELKTPLGDTTHT | CPRCP(EPKSCDTPPPCPRCP)₃ | APELLGGP |
| *IgG4* | ESKYG . . . . PP | CPSCP | APPEFLGGP |

The start-points and endpoints of the hinge domains and parts thereof (upper, core, lower) are determined by alignment of sequence with the above indicated wild-type sequence, by conventional methods of sequence alignment. In case of doubt, the start-point of the upper hinge is the first amino acid after position 215 (Eu numbering, wherein 215 typically is V) and the end-point of the upper hinge is the position before the first cysteine residue (of the core hinge domain) that forms a disulfide bridge with the hinge domain of the other heavy chain. Furthermore, in case of doubt, the start-point of the core hinge is the first cysteine residue that forms a disulfide bridge with the hinge domain of the other heavy chain and the end-point of the core hinge domain is the residue before residue 231 of the lower hinge domain (Eu numbering, wherein 231 typically is A).

The term "CH1 region" or "CH1 domain" as used herein is intended to refer to the CH1 region of an immunoglobulin heavy chain. Thus, for example the CH1 region of a human IgG1 antibody corresponds to amino acids 118-215 according to the Eu numbering. However, the CH1 region may also be any of the other subtypes as described herein.

The term "CH2 region" or "CH2 domain" as used herein is intended to refer to the CH2 region of an immunoglobulin heavy chain. Thus, for example the CH2 region of a human IgG1 antibody corresponds to amino acids 239-340 according to the EU numbering. However, the CH2 region may also be any of the other subtypes as described herein.

The term "CH3 region" or "CH3 domain" as used herein is intended to refer to the CH3 region of an immunoglobulin heavy chain. Thus, for example the CH3 region of a human IgG1 antibody corresponds to amino acids 341-447 according to the EU numbering. However, the CH3 region may also be any of the other subtypes as described herein or contain truncations such as absence of the C-terminal K residue.

Reference to amino acid positions in the Fc region/Fc domain in the present invention is according to the EU-numbering (Edelman et al., Proc Natl Acad Sci U S A. 1969 May;63(1):78-85; Kabat et al., Sequences of proteins of immunological interest. 5th Edition - 1991 NIH Publication No. 91-3242). An overview of amino acid numbering for IgG1, IgG2, IgG3 and IgG4 is provided in: https://www.imgt.org/IMGTScientificChart/Numbering/Hu_IGHGnber.html.

As indicated above, the term antibody as used herein, unless otherwise stated or clearly contradicted by context, includes fragments of an antibody that retain the ability to specifically bind to the antigen. It has been shown that the antigen-binding function of an antibody may be performed by fragments of a full-length antibody. Examples of binding fragments encompassed within the term "antibody" include, for example, a F(ab')2 fragment, i.e. a bivalent fragment comprising two Fab fragments linked by a disulfide bridge at the hinge region; Furthermore, although the two domains of the Fv fragment, VL and VH, are coded for by separate genes, they may be joined, using recombinant methods, by a synthetic linker that enables them to be made as a single protein chain in which the VL and VH domains pair to form monovalent molecules (known as single chain antibodies or single chain Fv (scFv), see for instance Bird et al., Science 242, 423-426 (1988) and Huston et al., PNAS USA 85, 5879-5883 (1988)). Such single-chain antibodies are encompassed within the term antibody unless otherwise indicated by context. The term antibody, unless specified otherwise, also includes polyclonal antibodies, monoclonal antibodies (mAbs), chimeric antibodies and humanized antibodies, and antibody fragments provided by any known technique, such as enzymatic cleavage, peptide synthesis and recombinant techniques.

In some embodiments, the first polypeptide or the second polypeptide, or both, comprise an antigen-binding region that is a single-domain antibody. Single-domain antibodies (sdAb, also called Nanobody^{®}, or VHH) are well known to the skilled person, see e.g. Hamers-Casterman et al. (1993) Nature 363:446, Roovers et al. (2007) Curr Opin Mol Ther 9:327 and Krah et al. (2016) Immunopharmacol Immunotoxicol 38:21. Single-domain antibodies comprise a single CDR1, a single CDR2 and a single CDR3.

The term "isotype" as used herein, refers to the immunoglobulin (sub)class (for instance IgG1, IgG2, IgG3, IgG4, IgD, IgA, IgE, or IgM) or any allotype thereof, such as IgG1m(za) and IgG1m(f) that is encoded by heavy chain constant region genes. Each heavy chain isotype can be combined with either a kappa (κ) or lambda (λ) light chain. An antibody in this invention can possess any isotype.

The term "parent antibody", is to be understood as an antibody which is identical to an antibody according to the invention, but wherein the parent antibody does not have one or more of the specified mutations. A "variant" or "antibody variant" is an antibody molecule which comprises one or more mutations, i.e. differences (substitutions, deletions and/or insertions) as compared to a "parent antibody" or reference antibody. Amino acid substitutions may exchange a native amino acid for another naturally-occurring amino acid, or for a non-naturally-occurring amino acid derivative. The amino acid substitution may be conservative or non-conservative. In the context of the present invention, conservative substitutions may be defined by substitutions within the classes of amino acids reflected in one or more of the following three tables:

### Amino acid residue classes for conservative substitutions

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

### Alternative conservative amino acid residue substitution classes

| | | | |
|---|---|---|---|
| 1 | A | S | T |
| 2 | D | E | |
| 3 | N | Q | |
| 4 | R | K | |
| 5 | I | L | M |
| 6 | F | Y | W |

### Alternative Physical and Functional Classifications of Amino Acid Residues

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | Q, T, K, S, G, N, D, E, and R |

In the context of the present invention, a substitution in a variant is indicated as:
Original amino acid - position - substituted amino acid;

The three-letter code, or one letter code, are used, including the codes Xaa and X to indicate amino acid residue. Accordingly, the notation "T366W" means that the variant comprises a substitution of threonine with tryptophan in the variant amino acid position corresponding to the amino acid in position 366 in the parent antibody.

Furthermore, the term "a substitution" includes a substitution into any one of the other nineteen natural amino acids, or into other amino acids, such as non-natural amino acids. For example, a substitution of amino acid T in position 366 includes each of the following substitutions: 366A, 366C, 366D, 366G, 366H, 366F, 366I, 366K, 366L, 366M, 366N, 366P, 366Q, 366R, 366S, 366E, 366V, 366W, and 366Y.

The term "full-length antibody" when used herein, refers to an antibody which contains all heavy and light chain constant and variable domains corresponding to those that are normally found in a wild-type antibody of that isotype.

The term "multispecific antibody" refers to an antibody having specificities for at least two different, such as at least three, typically non-overlapping, epitopes, due to the presence of two or more antigen-binding regions. Such epitopes may be on the same or on different target antigens. If the epitopes are on different targets, such targets may be on the same cell or different cells or cell types.

The term "bispecific antibody" refers to an antibody having specificities for two different, typically non-overlapping, epitopes, due to the presence of two antigen-binding regions. Such epitopes may be on the same or different targets. If the epitopes are on different targets, such targets may be on the same cell or different cells or cell types.

Examples of different classes of bispecific or multispecific antibodies include but are not limited to (i) IgG-like molecules with complementary CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to an extra Fab fragment or parts of a Fab fragment; (iv) Fc fusion molecules, wherein single chain Fv molecules or stabilized diabodies are fused to heavy-chain constant- domains, Fc-regions or parts thereof; (v) Fab fusion molecules, wherein different Fab fragments are fused together, fused to heavy-chain constant-domains, Fc-regions or parts thereof; and (vi) scFv-and diabody-based and heavy chain-based antibodies (e.g., domain antibodies, Nanobodies^{®}) wherein different single chain Fv molecules or different diabodies or different heavy-only chain antibodies (e.g. domain antibodies, Nanobodies^{®}) are fused to each other or to another protein or carrier molecule fused to heavy-chain constant-domains, Fc-regions or parts thereof, (vii) IgG-like molecules with symmetric or asymmetric architecture.

Examples of IgG-like molecules with complementary CH3 domains molecules include but are not limited to the Triomab^{®} (Trion Pharma/Fresenius Biotech), the Knobs-into-Holes (Genentech), CrossMAbs (Roche) and the electrostatically-matched (Amgen, Chugai, Oncomed), the LUZ-Y (Genentech, Wranik et al. J. Biol. Chem. 2012, 287(52): 43331-9, doi: 10.1074/jbc.M112.397869. Epub 2012 Nov 1), DIG-body and PIG-body (Pharmabcine, WO2010134666, WO2014081202), the Strand Exchange Engineered Domain body (SEEDbody)(EMD Serono), the Biclonics (Merus, WO2013157953), FcΔAdp (Regeneron), bispecific IgG1 and IgG2 (Pfizer/Rinat), Azymetric scaffold (Zymeworks/Merck,), mAb-Fv (Xencor), bivalent bispecific antibodies (Roche, WO2009080254) and DuoBody^{®} molecules (Genmab, WO11131746).

Examples of recombinant IgG-like dual targeting molecules include, but are not limited to, Dual Targeting (DT)-Ig (GSK/Domantis, WO2009058383), Two-in-one Antibody (Genentech, Bostrom, et al 2009. Science 323, 1610-1614), Cross-linked Mabs (Karmanos Cancer Center), mAb2 (F-Star), Zybodies^{™} (Zyngenia, LaFleur et al. MAbs. 2013 Mar-Apr;5(2):208-18), approaches with common light chain (reviewed in Labrijn et al. (2019) Nat Rev Drug Discov 18(8): 585), KλBodies (NovImmune, WO2012023053), US2020319181 (Merus) and CovX-body^{®} (CovX/Pfizer, Doppalapudi, V.R., et al 2007. Bioorg. Med. Chem. Lett. 17,501-506).

Examples of IgG fusion molecules include but are not limited to Dual Variable Domain (DVD)-Ig (Abbott), Dual domain double head antibodies (Unilever; Sanofi Aventis), IgG-like Bispecific (ImClone/Eli Lilly, Lewis et al. Nat Biotechnol. 2014 Feb;32(2):191-8), Ts2Ab (MedImmune/AZ, Dimasi et al. J Mol Biol. 2009 Oct 30;393(3):672-92) and BsAb (Zymogenetics, WO2010111625), HERCULES (Biogen Idec), scFv fusion (Novartis), scFv fusion (Changzhou Adam Biotech Inc) and TvAb (Roche).

Examples of Fc fusion molecules include but are not limited to scFv/Fc Fusions (Academic Institution, Pearce et al Biochem Mol Biol Int. 1997 Sep;42(6):1179), SCORPION (Emergent BioSolutions/Trubion, Blankenship JW, et al. AACR 100th Annual meeting 2009 (Abstract #5465); Zymogenetics/BMS, WO2010111625), Dual Affinity Retargeting Technology (Fc-DARTTM) (MacroGenics) and Dual(ScFv)2-Fab (National Research Center for Antibody Medicine - China).

Examples of Fab fusion bispecific antibodies include but are not limited to F(ab)2 (Medarex/AMGEN), Dual-Action or Bis-Fab (Genentech), Dock-and-Lock^{®} (DNL) (ImmunoMedics), Bivalent Bispecific (Biotecnol) and Fab-Fv (UCB-Celltech).

Examples of scFv-, diabody-based and domain antibodies include but are not limited to Bispecific T Cell Engager (BiTE^{®}) (Micromet, Tandem Diabody (Tandab) (Affimed), Dual Affinity Retargeting Technology (DARTTM) (MacroGenics), Single-chain Diabody (Academic, Lawrence FEBS Lett. 1998 Apr 3;425(3):479-84), TCR-like Antibodies (AIT, ReceptorLogics), Human Serum Albumin ScFv Fusion (Merrimack, WO2010059315) and COMBODY molecules (Epigen Biotech, Zhu et al. Immunol Cell Biol. 2010 Aug;88(6):667-75), dual targeting nanobodies^{®} (Ablynx, Hmila et al., FASEB J. 2010), dual targeting heavy chain only domain antibodies.

In the context of antibody binding to an antigen, the terms "binds" or "specifically binds" refer to the binding of an antibody to a predetermined antigen or target to which binding typically is with an affinity corresponding to a K_{D} of about 10⁻⁴ M or less, such about 10⁻⁵ M or less, 10⁻⁶ M or less, e.g. 10⁻⁷ M or less, such as about 10⁻⁸ M or less, such as about 10⁻⁹ M or less, about 10⁻¹⁰ M or less, or about 10⁻¹¹ M or even less, e.g. when determined as described in the Examples herein. K_{D} values can for example be determined using for instance surface plasmon resonance (SPR) technology in a BIAcore T200 or bio-layer interferometry (BLI) in an Octet RED96 instrument using the antigen as the ligand and the binding moiety or binding molecule as the analyte. Specific binding means that the antibody binds to the predetermined antigen with an affinity corresponding to a K_{D} that is at least ten-fold lower, such as at least 100-fold lower, for instance at least 1,000 fold lower, such as at least 10,000 fold lower, for instance at least 100,000 fold lower than its affinity for binding to a non-specific antigen (e.g., BSA, casein) other than the predetermined antigen or a closely-related antigen. The degree with which the affinity is lower is dependent on the K_{D} of the binding moiety or binding molecule, so that when the K_{D} of the binding moiety or binding molecule is very low (that is, the binding moiety or binding molecule is highly specific), then the degree with which the affinity for the antigen is lower than the affinity for a non-specific antigen may be at least 10,000-fold. The term "K_{D}" (M), as used herein, refers to the dissociation equilibrium constant of a particular interaction between the antigen and the binding moiety or binding molecule.

The terms "first" and "second" when used herein in connection with polypeptides, such as antibody domains, do not refer to their orientation / position in the antibody, i.e. they have no meaning with regard to the N- or C-terminus. The terms also do not necessarily imply that the "first" and "second" domains are different from each other. In some embodiments, they may be identical. The terms "first" and "second" only serve to correctly and consistently refer to the two antigen-binding regions in the claims and the description.

The term "tag" has its usual meaning in the art and refers to a moiety for binding or recognition that is linked to a polypeptide. In the present context, a tag is typically a peptide moiety of less than 20 or 15 amino acids in length, e.g. 1, 2, 3, 4, 5, 6, 7, 8, 9 or 10 amino acids in length. Tags used in the present invention comprise histidine residues or functional equivalents thereof.

"% sequence identity", when used herein, refers to the number of identical nucleotide or amino acid positions shared by different sequences (i.e., % identity = # of identical positions/total # of positions x 100), taking into account the number of gaps, and the length of each gap, which need to be introduced for optimal alignment. The percent identity between two nucleotide or amino acid sequences may e.g. be determined using the algorithm of E. Meyers and W. Miller, Comput. Appl. Biosci 4, 11-17 (1988) which has been incorporated into the ALIGN program (version 2.0), using a PAM120 weight residue table, a gap length penalty of 12 and a gap penalty of 4.

Polypeptides, such as antibodies, are typically produced recombinantly, i.e. by expression of nucleic acid constructs encoding the antibodies in suitable host cells, followed by purification of the produced recombinant antibody from the cell culture. Nucleic acid constructs can be produced by standard molecular biological techniques well-known in the art. The constructs are typically introduced into the host cell using an expression vector. Suitable nucleic acid constructs and expression vectors are known in the art. Host cells suitable for the recombinant expression of antibodies are well-known in the art, and include CHO (Chinese Hamster Ovary), HEK-293, Expi293F, PER-C6, NS/0 and Sp2/0 cells.

As described herein, in a first aspect, the invention relates to a method for producing a heterodimeric polypeptide composition, said heterodimeric polypeptide comprising a first polypeptide and a second polypeptide,
wherein said first polypeptide and said second polypeptide are different and are able to form heterodimers and homodimers,
and wherein the first polypeptide comprises an N- or C-terminal histidine-based tag that more efficiently binds to a histidine affinity resin when present on the termini of both polypeptides (i.e. on both N-termini or both C-termini) of a dimer than when present on the terminus of only one polypeptide of a dimer, said method comprising:
   a) providing a composition comprising heterodimers of the first and second polypeptides and homodimers of the first polypeptide, preferably by:
      (i) co-expressing said first polypeptide and second polypeptides in a single host cell, wherein preferably the first polypeptide is overexpressed relative to the second polypeptide, and collecting supernatant,
         or
      (ii) expressing said first polypeptide and second polypeptide in separate host cells, collecting, optionally performing a purification step, and mixing supernatants, wherein preferably the first polypeptide is added in excess, and performing an exchange reaction to obtain heterodimers,
   b) contacting said composition with a histidine affinity resin and allowing homodimers of the first polypeptide to preferentially bind to the resin, and
   c) recovering the unbound fraction to obtain a composition that is enriched for heterodimers.

In some embodiments, the composition comprising heterodimers of the first and second polypeptides and homodimers of the first polypeptide may further comprise homodimers of the second polypeptide, but preferably the proportion of homodimers of the second polypeptide is reduced or elimination by overexpression or addition in excess of the first polypeptide.

In some embodiments, said method comprises providing a composition comprising heterodimers of the first and second polypeptides and homodimers of the first polypeptide by co-expressing said first polypeptide and second polypeptides in a single host cell.

The first polypeptide may comprise a first Fc region and/or the second polypeptide may comprise a second Fc region.

In some embodiments, the first polypeptide comprises a first Fc region and the second polypeptide comprises a second Fc region and the first and second Fc regions comprise asymmetric amino acid mutations that favor the formation of heterodimers over the formation of homodimers, wherein preferably:
(i) the first Fc region comprises a T366W substitution and the second Fc region comprises T366S, L368A and Y407V substitutions, or vice versa,
(ii) the first Fc region comprises a F405L substitution and the second Fc region comprises a K409R substitution, or vice versa, or
(iii) the first Fc region comprises the substitutions F405L and R409K and the second Fc region is wild-type at the 405 and 409 positions, or vice versa,
wherein the numbering is according to the EU numbering.

In some embodiments, the first polypeptide and the second polypeptide both comprise an antigen-binding region, each independently selected from a Fab region, an scFv and a VHH domain.

In some embodiments, the heterodimer is a full-length antibody, such as a full-length IgG1 antibody or a full-length IgG4 antibody.

In some embodiments, the first polypeptide comprises a first Fc region and the second polypeptide comprises a second Fc region and the Fc regions comprise a CPSC or CPAC sequence in the core hinge.

In some embodiments, the heterodimeric polypeptide is a multispecific antibody, such as a bispecific antibody or a trispecific antibody.

In some embodiments, the first and/or the second polypeptide is co-expressed with a light chain, wherein preferably both the first polypeptide and the second polypeptide are co-expressed with a light chain and wherein more preferably said light chains are identical.

In some embodiments, the histidine-based tag is located at the C-terminus of the first Fc region.

In some embodiments, the histidine-based tag comprises at least 2, 3, 4, 5, 6, 7, 8, 9, 10, 11 or 12 histidine residues, preferably at least or exactly 4, 5 or 6 consecutive histidine residues. The tagged molecule has an affinity for a histidine affinity resin which is substantially weaker (e.g., more than 10-fold weaker or in the range of 1.5-fold to 10-fold weaker, preferably 2-fold to 4-fold weaker) when containing a single N- or C-terminal tag compared to a molecule having two N- or C-terminal tags.

In some embodiments, the histidine-based tag is attached to the N- or C-terminus via a linker comprising amino acid residues, such as glycine residues.

In some embodiments, the second polypeptide does not comprise a histidine-based tag.

In some embodiments, the histidine affinity resin is a Ni-based or Co-based resin.

In some embodiments of the method, step b) is performed in a solution, preferably a buffer solution, comprising histidine or a functional equivalent thereof, such as a positively-charged natural or non-natural amino acid, imidazole, a chelator (e.g., EDTA) or other compounds that shield the affinity resin, or other compounds that interfere with histidine binding to the resin.

Preferably, the pH of the solution is above 6, such as above 6.5, for example between 6.5 and 8.5, such as between 7 and 7.5. In one embodiment, the buffer is phosphate-buffered saline. In some embodiments, the solution comprises at least 10 mM histidine, such as at least 20 mM histidine, for example at least 30 mM histidine, such as at least 40 mM histidine. In some embodiments, the solution comprises less than 100 mM, such as less than 50 mM histidine.

In some embodiments, the tag comprises 4 consecutive histidines and the solution comprises at least 10 mM histidine, such as at least 20 mM histidine, for example at least 30 mM histidine, such as at least 40 mM histidine. In some embodiments, the solution comprises less than 100 mM, such as less than 50 mM histidine.

In some embodiments, the tag comprises 5 consecutive histidines and the solution comprises at least 10 mM histidine, such as at least 20 mM histidine, for example at least 30 mM histidine, such as at least 40 mM histidine. In some embodiments, the solution comprises less than 100 mM, such as less than 50 mM histidine.

In some embodiments, the tag comprises 6 consecutive histidines and the solution comprises at least 10 mM histidine, such as at least 20 mM histidine, for example at least 30 mM histidine, such as at least 40 mM histidine. In some embodiments, the solution comprises less than 100 mM, such as less than 50 mM histidine.

In some embodiments, steps b) and c) may be performed in batch mode. In other embodiments, step b) and c) are performed using a column.

In some embodiments, the method comprises a further step of purifying the resulting composition using a protein A affinity column.

In some embodiments, the method comprises a further step of formulating the heterodimer-enriched composition obtained in step c) to obtain a pharmaceutical composition.

In some embodiments, the exchange reaction is a controlled Fab-arm exchange reaction (Labrijn et al. (2013) Proc Natl Acad Sci U S A. 110(13):5145-50).

In a further aspect, the invention relates to a method for producing a heterodimeric antibody composition, said heterodimeric antibody comprising a first heavy chain polypeptide and a second heavy chain polypeptide,
wherein said first heavy chain polypeptide and said second heavy chain polypeptide are different, and preferably bind different targets, and are able to form heterodimers and homodimers,
and wherein the first heavy chain polypeptide comprises an N- or C-terminal histidine-based tag that more efficiently binds to a histidine affinity resin when present on the termini of both heavy chain polypeptides of a dimer than when present on the terminus of only one heavy chain polypeptide of a dimer, said method comprising:
   a) providing a composition comprising heterodimers of the first and second heavy chain polypeptides and homodimers of the first heavy chain polypeptide, preferably by:
      (i) co-expressing said first heavy chain polypeptide and second heavy chain polypeptides, optionally together with one or more light chain polypeptides, in a single host cell, wherein preferably the first heavy chain polypeptide is overexpressed relative to the second heavy chain polypeptide, and collecting supernatant,
         or
      (ii) expressing said first heavy chain polypeptide and second heavy chain polypeptide, optionally together with one or more light chain polypeptides, in separate host cells, collecting and mixing supernatants, wherein preferably the first heavy chain polypeptide is added in excess, and performing an exchange reaction, such as a controlled Fab-arm exchange reaction, to obtain heterodimers,
   b) contacting said composition with a histidine affinity resin and allowing homodimers of the first heavy chain polypeptide to preferentially bind to the resin, and
   c) recovering the unbound fraction to obtain a composition that is enriched for bispecific antibodies.

In a further aspect, the invention relates to a composition obtainable by the method of the invention as described herein.

In an even further aspect, the invention relates to a method for producing and screening a library comprising a plurality of heterodimeric polypeptides, comprising performing the method according to the invention as described herein for a plurality of heterodimeric polypeptides in parallel and performing the further step of screening the plurality of compositions obtained in step c) for heterodimers having desired functional properties.

### EXAMPLES

### Example 1: Antibody constructs and production

Synthetic genes encoding VHH or VH, light chain regions (where appropriate) and IgG4 constant regions with asymmetric amino acid mutations, in this example with or without R409K and F405L substitutions and with or without a His-4 tag (G4-H4) or His-6 tag (G4-H6), were cloned into expression vectors using BamHI and Notl restriction enzymes and ligation.

HEK293E cells were transiently co-transfected with DNA plasmids using the rPEx technology (ImmunoPrecise Antibodies) and cultured in FreeStyle medium containing 0.5% Primatone for 6 days. Culture supernatant containing recombinant antibody was harvested by centrifugation followed by filtration over a 0.2 µm SartoClear bottle top filter.

### Example 2: HisMag Sepharose bead purification

Nickel-based magnetic HisMag Sepharose excel beads (5 or 10 µL settled beads, Cytiva), equilibrated in PBS, were added to 1.5 mL Eppendorf tubes containing 500 µL sterile filtered culture supernatants (containing recombinant antibody). Beads and supernatant were then diluted 1:1 to a final volume of 1 mL with PBS containing L-histidine and incubated on a roller bench and samples were taken at 2, 4, 16 and/or 72 hour time points. Beads were captured using a MagRack6 (Cytiva).

### Example 3: Analysis of antibody heterodimer enrichment at various L-histidine concentrations

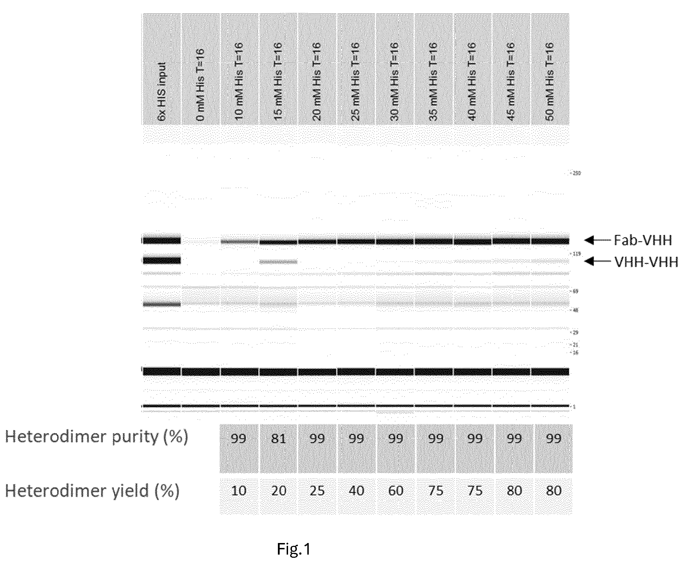

HEK293E cells were transfected with a plasmid encoding the heavy chain of an antibody that binds a cell-surface receptor (abbreviated: CSR), a plasmid encoding the light chain of said antibody, and a plasmid encoding a fusion protein of a gp120 specific VHH, an IgG4 Fc (hinge-CH2-CH3) region and a C-terminal tag comprising six consecutive histidine residues (G4-H6) (SEQ ID NO:12 GGGGHHHHHH) at a 0.2 : 0.2 : 0.6 ratio and cultured as described above. Culture supernatants were subjected to HisMag Sepharose bead purification as described above and samples were taken at 2, 4, 16 and/or 72 hour time points. The residual samples - after magnetic bead capture - were analyzed by capillary electrophoresis under non-reducing conditions (LabChip GXII Touch, Revvity). Figure 1 shows an example of the analysis of the preparation of CSR-Fab-hIgG4-R409K-F405Lxgp120-VHH-hlgG4-G4-H6 using 5 µL settled beads, 16 hour roller bench incubation. The unpurified culture medium (left lane) contained both the FabxVHH heterodimer and the VHH-VHH homodimer. After purification, the samples were strongly enriched for heterodimers. With increasing L-histidine concentration present during bead purification, using 16 hour incubation with 5 µL settled beads, the homodimers bound more efficiently to the beads and thus, after removal of the beads, the samples were further enriched for heterodimers.

Table 1 shows the results of the analysis of all variations in the amount of beads, incubation time and L-histidine concentration for CSR-Fab-hIgG4-R409K-F405Lxgp120-VHH-hIgG4-G4-H4, Table 2 shows these results for CSR-Fab-hIgG4-R409K-F405Lxgp120-VHH-hIgG4-G4-H6. For this antibody heterodimer, purity up to 99% and yield of 80-85% was reached.

**Table 1: Analysis of the preparation of CSR-Fab-hIgG4-R409K-F405Lxgp120-VHH-hlgG4-G4-H4**

| 5 µl HisMag Sepharose excel beads | | | |
|---|---|---|---|
| Incubation time (h) | [L-histidine] (mM) | Heterodimer purity (%) | Heterodimer yield (%) |
| 2 | 5 | 80 | 40 |
| | 10 | 80 | 55 |
| | 15 | 85 | 60 |
| | 20 | 74 | 80 |
| | 25 | 74 | 80 |
| 4 | 5 | 99 | 40 |
| | 10 | 99 | 55 |
| | 15 | 99 | 60 |
| | 20 | 88 | 80 |
| | 25 | 87 | 85 |
| 16 | 5 | 99 | 40 |
| | 10 | 98 | 75 |
| | 15 | 96 | 75 |
| | 20 | 93 | 80 |
| | 25 | 83 | 85 |

| 10 µl HisMag Sepharose excel beads | | | |
|---|---|---|---|
| 2 | 5 | 96 | 8 |
| | 10 | 96 | 15 |
| | 15 | 94 | 30 |
| | 20 | 87 | 65 |
| | 25 | 86 | 65 |
| 4 | 5 | 99 | 20 |
| | 10 | 99 | 28 |
| | 15 | 96 | 66 |
| | 20 | 96 | 70 |
| | 25 | 94 | 70 |
| 72 | 5 | 99 | 20 |
| | 10 | 99 | 50 |
| | 15 | 97 | 66 |
| | 20 | 94 | 70 |
| | 25 | 94 | 75 |

**Table 2: Analysis of the preparation of CSR-Fab-hIgG4-R409K-F405Lxgp-120-VHH-hIgG4-G4-H6**

| 5 µl HisMag Sepharose excel beads | | | |
|---|---|---|---|
| Incubation time (h) | [L-histidine] (mM) | Heterodimer purity (%) | Heterodimer yield (%) |
| 2 | 10 | 72 | 10 |
| | 15 | 75 | 20 |
| | 20 | 76 | 30 |
| | 25 | 76 | 40 |
| | 30 | 76 | 55 |
| | 35 | 77 | 65 |
| | 40 | 75 | 70 |
| | 45 | 75 | 80 |
| | 50 | 76 | 80 |
| 4 | 10 | 99 | 10 |
| | 15 | 99 | 20 |
| | 20 | 99 | 30 |
| | 25 | 95 | 40 |
| | 30 | 95 | 55 |
| | 35 | 90 | 65 |
| | 40 | 88 | 67 |
| | 45 | 86 | 80 |
| | 50 | 85 | 80 |
| 16 | 10 | 99 | 10 |
| | 15 | 81 | 20 |
| | 20 | 99 | 25 |
| | 25 | 99 | 40 |
| | 30 | 99 | 60 |
| | 35 | 99 | 75 |
| | 40 | 99 | 75 |
| | 45 | 99 | 80 |
| | 50 | 99 | 80 |

| 10 µl HisMag Sepharose excel beads | | | |
|---|---|---|---|
| 2 | 10 | 99 | 10 |
| | 15 | 99 | 10 |
| | 20 | 99 | 15 |
| | 25 | 99 | 15 |
| | 30 | 92 | 20 |
| | 35 | 92 | 25 |
| | 40 | 92 | 40 |
| | 45 | 91 | 50 |
| | 50 | 85 | 55 |
| 4 | 10 | 72 | 10 |
| | 15 | 99 | 10 |
| | 20 | 99 | 15 |
| | 25 | 99 | 20 |
| | 30 | 99 | 20 |
| | 35 | 99 | 35 |
| | 40 | 99 | 40 |
| | 45 | 99 | 50 |
| | 50 | 99 | 55 |
| 72 | 10 | 99 | 10 |
| | 15 | 99 | 10 |
| | 20 | 99 | 20 |
| | 25 | 99 | 30 |
| | 30 | 99 | 50 |
| | 35 | 99 | 70 |
| | 40 | 99 | 70 |
| | 45 | 99 | 77 |
| | 50 | 99 | 80 |

### Example 4: Analysis of antibody heterodimer enrichment of antibodies in high throughput

HEK293E cells were transfected with a plasmid encoding a VH of an antibody recognizing a cell surface receptor (cell surface receptor A, CSR-A) with a IgG4-R409K-F405L constant domain, a plasmid encoding the light chain (LC) of said antibody and several plasmids encoding different VHHs binding a second cell surface receptor (cell surface receptor B, CSR-B) linked via a GS-linker (2 aa 'GS') to VHHs binding a third cell surface receptor (cell surface receptor C, CSR-C) with an IgG4 constant domain containing a C-terminal His tag. In pilot experiments the transfection ratio of the plasmids, the length of the his tag and the amount of beads were optimized for a high throughput production set-up.

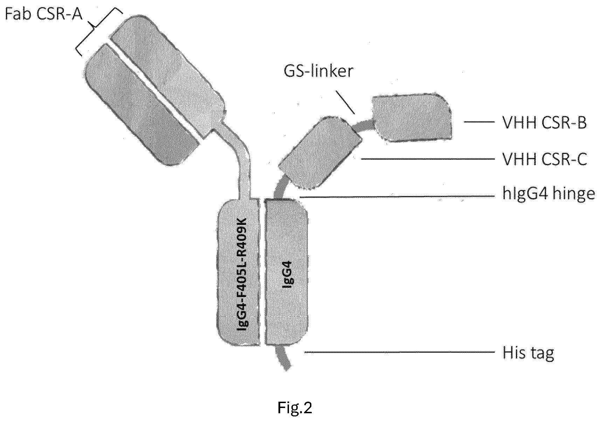

Figure 2 shows a schematic representation of the resulting antibody heterodimer.

A VH:LC:VHH-VHH plasmid ratio of 1:1:3 and His-6 tag (SEQ ID NO:12) was used in a high throughput production campaign. The CSR-A VH (with IgG4- R409K-F405L constant domain) and LC were combined with 99 different CSR-B VHH-CSR-C VHH (with an IgG4 constant domain containing the C-terminal 6-His tag) combinations. Culture supernatants were subjected to HisMag Sepharose bead purification essentially as described above using 1.0 µL beads per 100 µL production supernatant, 35 mM L-histidine and 12 hours incubation. The residual samples - after magnetic bead capture - were analyzed by capillary electrophoresis as described above.

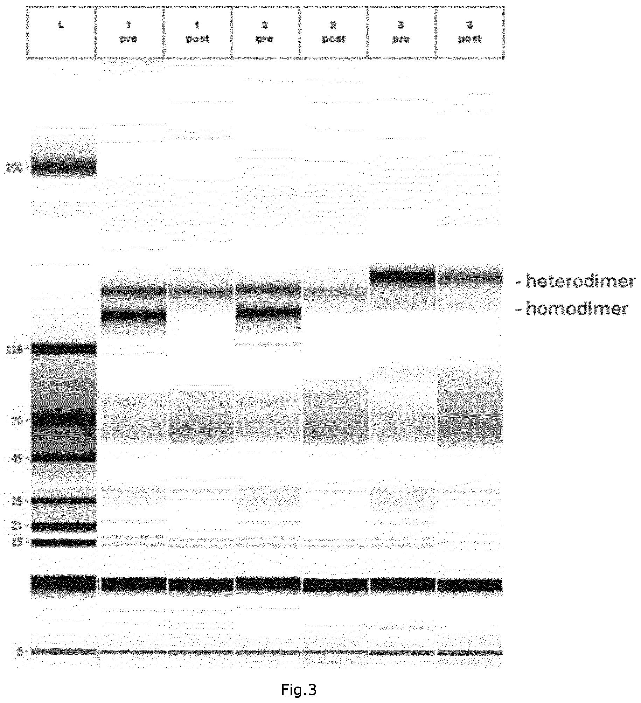

Of the productions, 89 of the 99 yielded sufficient amounts of antibody (before and after purification) for LabChip analysis. This analysis showed that after HisMag Sepharose bead purification almost entirely pure antibody heterodimer compositions were obtained for all purifications, with a heterodimer yield of 44 ± 27 % (mean ± standard deviation). Figure 3 shows the analysis of 3 examples of the produced antibody preparations before and after purification.

## Claims

1. A method for producing a heterodimeric polypeptide composition, said heterodimeric polypeptide comprising a first polypeptide and a second polypeptide,
wherein said first polypeptide and said second polypeptide are different and are able to form heterodimers and homodimers,
and wherein the first polypeptide comprises an N- or C-terminal histidine-based tag that more efficiently binds to a histidine affinity resin when present on the termini of both polypeptides of a dimer than when present on the terminus of only one polypeptide of a dimer, said method comprising:
a) providing a composition comprising heterodimers of the first and second polypeptides and homodimers of the first polypeptide, preferably by:
(i) co-expressing said first polypeptide and second polypeptides in a single host cell, wherein preferably the first polypeptide is overexpressed relative to the second polypeptide, and collecting supernatant,
or
(ii) expressing said first polypeptide and second polypeptide in separate host cells, collecting and mixing supernatants, wherein preferably the first polypeptide is added in excess, and performing an exchange reaction to obtain heterodimers,
b) contacting said composition with a histidine affinity resin and allowing homodimers of the first polypeptide to preferentially bind to the resin, and
c) recovering the unbound fraction to obtain a composition that is enriched for heterodimers.

2. The method according to claim 1, wherein said method comprises providing a composition comprising heterodimers of the first and second polypeptides and homodimers of the first polypeptide by co-expressing said first polypeptide and second polypeptides in a single host cell.

3. The method according to claim 1 or 2, wherein the first polypeptide comprises a first Fc region and the second polypeptide comprises a second Fc region.

4. The method according to claim 3, wherein the first and second Fc regions comprise asymmetric amino acid mutations that favor the formation of heterodimers over the formation of homodimers, wherein preferably:
(iv)the first Fc region comprises a T366W substitution and the second Fc region comprises T366S, L368A and Y407V substitutions, or vice versa,
(v) the first Fc region comprises a F405L substitution and the second Fc region comprises a K409R substitution, or vice versa, or
(vi) the first Fc region comprises the substitutions F405L and R409K and the second Fc region is wild-type at the 405 and 409 positions, or vice versa,
wherein the numbering is according to the EU numbering.

5. The method according to any one of the preceding claims, wherein the first polypeptide and the second polypeptide both comprise an antigen-binding region, independently selected from a Fab region, an scFv and a VHH domain.

6. The method according to any one of the preceding claims, wherein the heterodimer is a full-length antibody, such as a full-length IgG1 antibody or a full-length IgG4 antibody.

7. The method according to any one of the preceding claims, wherein the Fc region comprises a CPSC or CPAC sequence in the core hinge.

8. The method according to any one of the preceding claims, wherein the heterodimeric polypeptide is a multispecific antibody, such as a bispecific antibody or a trispecific antibody.

9. The method according to any one of the preceding claims, wherein the first and/or the second polypeptide is co-expressed with a light chain, wherein preferably both the first polypeptide and the second polypeptide are co-expressed with a light chain and wherein more preferably said light chains are identical.

10. The method according to any one of the preceding claims, wherein the histidine-based tag is located at the C-terminus of the first Fc region.

11. The method according to any one of the preceding claims, wherein the histidine-based tag comprises at least 2, 3, 4, 5, 6, 7, 8, 9, 10, 11 or 12 histidine residues, preferably at least or exactly 4, 5 or 6 consecutive histidine residues.

12. The method according to any one of the preceding claims, wherein the second polypeptide does not comprise a histidine-based tag.

13. The method according to any one of the preceding claims, wherein the histidine affinity resin is a Ni-based or Co-based resin.

14. The method according to any one of the preceding claims, wherein step b) is performed in a solution, preferably a buffer solution, comprising histidine or a functional equivalent thereof.

15. A method for producing and screening a library comprising a plurality of heterodimeric polypeptides, comprising performing the method according to any one of claims 1 and 14 for a plurality of heterodimeric polypeptides in parallel and performing the further step of screening the plurality of compositions obtained in step c) for heterodimers having desired functional properties.
